# EUROPEAN PATENT APPLICATION

(11) **EP 4 763 205 A1**
(43) Date of publication of application: **24.06.2026**
(21) Application number: 24221029.2
(22) Date of filing: 18.12.2024
(51) Int. Cl.: A61K 31/40, A61P 17/02, A61K 9/16, A61K 9/50

(54) **STATIN-CONTAINING COMPOSITION FOR TREATMENT OF WOUNDS**

(71) Applicant: Paul Hartmann AG, 89522 Heidenheim (DE)
(72) Inventor: Brückner, Katerina, 612 00 Brno (CZ); Horavová, Hana, 612 00 Palackého trída 1946/1 (CZ); Vyslouzil, Jakub, 612 00 Brno (CZ); Smola, Hans, 89522 Heidenheim (DE)
(74) Representative: Ter Meer Steinmeister & Partner

(57) **Abstract**

The invention essentially relates to statin for use in the treatment of a wound with impaired wound healing.

## Description

The invention essentially relates to statin for use in the treatment of a wound with impaired wound healing.

### Technical Background

A wound can be regarded as the separation of the contiguity of tissues of the skin, wherein this can be combined with loss of substance. Wounds can *inter alia* be characterized by the kind of impact, their size and the state of the healing.

The healing of wounds is based on the ability of the skin to regenerate tissue, such as epithelial tissue, connective and supporting tissue. The regeneration of tissue is a complex occurrence of cell activities overlapping each other, wherein said cell activities promote the healing process step by step. According to the literature, there are four main phases of healing. In the first phase, the hemostasis phase, the bleeding is stopped, and thrombin initiates the formation of a fibrin mesh. In the second phase, the defensive/inflammatory phase, specific white blood cells and macrophages successively enter the wound to destroy bacteria and remove debris. In addition, growth factors are provided to attract immune system cells to the wound and to facilitate tissue repair. The third phase is the proliferative phase. After the cleaning of the wound, said phase is to fill and cover the wound. During wound filling, granulation tissue fills the wound bed with connective tissue, and new blood vessels are formed. Subsequently, the wound margins contract and pull towards the wound center. In the last stage of said phase, epithelial cells are formed which cover the wound with epithelium. The fourth phase is the maturation phase. In said phase, the new tissue matures, i.e. it becomes stronger and more flexible with the reorganization of collagen fibers.

Statins are essentially used in the art as orally administrable compounds for reducing the lipid and/or cholesterol level in patients. The corresponding compositions comprise dosage forms like tablets. It is reported that orally administered statins could also have effects on wound healing. For example, by inhibiting the 3-hydroxy-3-methylglutaryl coenzyme-A-reductase (shortly referred to as HMG CoA reductase), which is responsible for the lipid/cholesterol-lowering effect, statin promotes the neovascularization of ischemic tissue, which in turn might be advantageous for wound healing.

In particular WO 2010/127313 A1 suggests that wound healing might be improved by a transdermal application of statins, i.e. statins are administered via healthy skin.

Furthermore, it was reported that statins could be applied in the form of creams or ointments. For example, US 2022/0133743 A1 describes a composition of statins and cholesterol and pharmaceutically acceptable carrier for the reduction of scar formation, wherein the dosage form can preferably be an emulsion or microemulsion.

However, the wound healing properties of the systems suggested in the prior art are still improvable in view of efficacy and in view of risk of traumas caused by the necessary changes of the wound dressings due to the loss of fast-releasing statin and the consequent loss of efficiency.

Hence, it is an object of the present invention to provide a therapeutic use and treatment methods which overcome the shortcomings of the prior art, since there is still a need for compounds for use in a method for treating wounds, in particular poorly healing wounds.

In particular, therapeutic use of a compound should be provided which enables advantageous wound healing. In particular the therapeutic use should provide an advantageous reduction of scar formation.

Further, a therapeutic use should be provided which enables that a dressing change has to be carried out only after a longer period of time, such as 2-3 days.

Moreover, a therapeutic use for treating poorly healing wounds, such as chronic and/or ischemic wounds, should be provided.

Further, a therapeutic use should be provided which achieves a high local concentration of statin at the wound, preferably wherein the disadvantages associated with systemic application should be reduced or preferably completely avoided. Said concentration should be achieved rapidly and maintained over a long period.

Hence, it was an object of the present invention to overcome at least one of the above problems.

### Summary of the invention

The present invention is suitable to solve one or more of the above-listed objectives by a statin which is used in the treatment of a wound, wherein a first solid statin-containing composition is applied directly to the wound and provides modified release of statin.

Thus, the subject of the present invention is statin for use in the treatment of a wound with impaired healing, wherein a first solid statin-containing composition is applied directly into the wound and wherein said first composition provides modified release of statin.

Further, the present invention is directed to a method for treating a wound with impaired healing comprising applying statin directly into the wound, wherein statin contained in a first solid composition and wherein said first composition provides modified release of statin.

### Figures:

Figure 1: Release of atorvastatin from present granulates inter alia containing Eudragit^{®} RS and Eudragit^{®} L in different ratios
Figure 2: Release of atorvastatin from a mixture of present granulate and crystalline atorvastatin (15% of total amount of atorvastatin)
Figure 3: Release of atorvastatin from a mixture of present granulate and crystalline atorvastatin (30% of total amount of atorvastatin)
Figure 4: Arginase Activity upon atorvastatin supplementation

### Detailed description

The following definitions are relevant in connection with the embodiments of the present invention.

The meaning of the terms "comprising" and "containing" is to be interpreted as encompassing all the specifically mentioned features as well as optional, additional unspecified ones, whereas the term "consisting of" only includes those features as specified. Therefore, "comprising" includes as a limiting case the meaning of "consisting of".

In this application the term "about" may be understood as +/- 5% of the corresponding value.

The terms "% by weight", "w/w" or "wt.-%" is understood to refer to the weight amount of the component relative to the total weight of the corresponding composition if not specified otherwise.

The meaning of the term "alkyl" is to be interpreted to encompass linear, branched and cyclic alkyl residues. Thus, for example an "alkyl having 1 to 3 carbon atoms" encompasses methyl, ethyl, propyl, isopropyl and cyclopropyl.

The present invention is directed to statin for use in the treatment of a wound with impaired healing.

A wound with impaired healing can be regarded as a wound that occurs in a person who has physiological impairments to healing. These can for example be a consequence of an infection, diabetes such as diabetes mellitus, venous insufficiencies such as venous reflux, arterial insufficiencies such as arterial insufficiency cell disease, steroid use, and/or exposure to chemotherapy, radiation and/or pressure. These pathophysiologic processes predispose wounds to deviate from the characteristics of acute wound healing. A wound with impaired wound healing can also be referred to as chronic wound.

The first solid statin-containing composition is applied directly into the wound. Such a kind of administration can also be regarded as topical administration. Contrary to systemic administration, wherein a composition is administered systemically for example via a tablet or an infusion and thus remote from the site where the therapeutic effect should be achieved, the first solid statin-containing composition granulate is administered topically, preferably it is administered directly to the site, i.e. the wound, where the therapeutic effect should take place.

The first composition is in solid form, i.e. the first composition is a solid composition. In line with the present application a solid composition refers to solid compositions at a temperature of 23°C and a pressure of about 101325 Pa (1 bar).

In a preferred embodiment the first statin-containing composition contains statin in combination with further pharmaceutically active agent(s). More preferred, the first statin-containing composition contains statin as the sole pharmaceutically active agent.

In a preferred embodiment according to the invention, the treatment comprises stimulation of the activation of macrophages in the wound and/or angiogenesis and/or epithelialization.

Macrophages can be regarded as scavenger cells (phagocytes) and leukocytes (white blood cells), i.e. cells of the immune system. They are used to eliminate microorganisms by phagocytosis and are probably the oldest parts of the innate immune defense in phylogenetic terms.

Angiogenesis can refer to a physiological process through which new blood vessels from pre-existing vessels are formed. Angiogenesis continues the growth of the vasculature mainly by processes of sprouting and splitting, but other processes such as coalescent angiogenesis, vessel elongation and vessel cooption can also play a role.

Epithelialization is the overgrowth of epithelial cells in a wound. It originates from intact epithelial tissue in the area of the wound edges. Epithelialization is the final phase of wound healing and follows granulation (formation of cell-rich connective tissue).

In a preferred embodiment the first solid statin-containing composition is in granular form, i.e. in form of a granulate. It is preferred that the granulate comprises (a) particles containing statin and release-modifying polymer and (b) binder.

Statin can be present in a crystalline or non-crystalline form.

The term "crystalline" can be used in the context of this invention to designate the state of solid substances in which the components (atoms, ions or molecules, i.e. in the case of crystalline statin the corresponding statin molecules) are arranged in an orderly repeating pattern, extending in all three spatial dimensions and thus exhibit a periodic arrangement over a great range (= long-range order).

In a preferred embodiment, statin contained in particles (a) is present in a non-crystalline form.

Generally, the term "non-crystalline" refers to any solid state being non-crystalline. Preferably, non-crystalline statin means statin in form of a solid dispersion or solid solution.

The term "non-crystalline" can be used in the context of this invention to designate the state of solid substances, in which the components (atoms, ions or molecules, i.e. in the case of non-crystalline statin the statin molecules) do not exhibit any periodic arrangement over a great range (= long-range order). In non-crystalline substances, the components are usually not arranged in a totally disordered fashion and completely randomly but are rather distributed in such a way that a certain regularity and similarity to the crystalline state can be observed with regard to the distance from and orientation towards their closest neighbours (= short-range order). Consequently, non-crystalline substances preferably possess a short-range order but no long-range order.

In contrast to anisotropic crystals, solid non-crystalline substances can be isotropic. Often, they do not have a defined melting point, but instead pass over into the liquid state after slowly softening. They can be distinguished from crystalline substances experimentally by means of X-ray diffraction, which does not reveal clearly defined interferences for them, but rather, in most cases, only a few diffuse interferences with small diffraction angles.

Non-crystalline statin may consist of pure non-crystalline statin. Alternatively, it may also contain small amounts of crystalline statin components, provided that no defined melting point of crystalline statin can be detected in a DSC. A mixture is preferred, containing 60 to 99.999% by weight of non-crystalline statin and 0.001 to 40% by weight of crystalline statin, more preferably the mixture contains 90 to 99.99% by weight of non-crystalline statin and 0.01 to 10% of crystalline statin, particularly preferably 95 to 99.9% by weight of non-crystalline statin and 0.1 to 5% of crystalline statin.

Apart from statin, particles (a) comprise a release-modifying polymer.

Release-modifying polymers are materials which are able to provide modified release properties, preferably due to their limited solubility, more preferably due to their limited solubility in aqueous conditions at pH 5.0. Preferably, the release-modifying polymer has a water solubility of less than 33 mg/l at a temperature of 25°C at a pH of 5.0, more preferably of less than 22 mg/l, still more preferably of less than 11 mg/l, especially from 0.01 to 5 mg/l. The water-solubility is determined according to the column elution method of the Dangerous Substances Directive (67/548/EEC), Annex V, Chapter A6. The pH value is determined according to Ph.Eur. 6.0, 2.2.3. The pH value of the aqueous medium usually is achieved by the addition of HCl (or NaOH) if necessary.

The solubility of the release-modifying polymer can be pH independent or pH dependent. Both embodiments are preferred. If the non-erodible material is pH dependent, it is preferred that the non-erodible material has a solubility in water at 25°C at a pH of 7.0 of more than 33 g/l, more preferably of 50 g/l to 10,000 g/l, still more preferably from 100 g/l to 5,000 g/l, in particular from 200 g/l to 2,000 g/l.

The release-modifying polymer can preferably have a weight average molecular weight ranging from 30.000 to 3,000,000 g/mol, preferably from more than 50,000 to 2,500,000 g/mol, more preferably from more than 125,000 to 2,000,000 g/mol, still more preferably from 250,000 to 2,200,000 g/mol, particularly preferred from 400,000 to 1,500,000 g/mol. Furthermore, a 2% w/w solution of the release-modifying polymer in water at pH 7.0 preferably has a viscosity of more than 2 mPas, more preferably of more than 5 mPas, particularly more than 8 mPas and up to 850 mPas, when measured at 25°C. The viscosity is determined according to Ph. Eur., 6^{th} edition, Chapter 2.2.10. In the above definition the term "solution" may also refer to a partial solution (in case that the polymer does not dissolve completely in the solution). The weight average molecular weight is preferably determined by gel electrophoresis.

The release-modifying polymer can preferably have a melting temperature of below 220°C, preferably between 25°C and 200°C. In a particularly preferred embodiment, the melting temperature is between 35°C and 190°C. The determination of the melting temperature is carried out according to Ph. Eur., 6^{th} edition, Chapter 2.2.15.

Release-modifying polymers are known in the art. Non-limiting examples of release-modifying polymers are cellulose and cellulose derivatives, acrylic polymers or acrylic copolymers, methacrylate (co)polymer, polyamide, polyethylene and polyethylene oxide, polyvinyl polymers, carnauba wax, cetyl alcohol, hydrogenated vegetable oils, microcrystalline waxes, monoglycerides, triglycerides and PEG monostearate; carbopol, gelatin and xanthan gum. Further suitable are chitosan, polylactic acid (PLA), polyglycolic acid (PGA), poly(lactic-co-glycolic) acid (PLGA), natural gum such as guar gum and tragacanth, polycaprolactones and polyorthoesters.

Cellulose and cellulose derivatives, polyvinyl polymers, acrylic polymers or acrylic copolymers and mixtures of one or more of these polymers are preferred as release-modifying polymer.

Cellulose and its derivatives are known in the art. Non-limiting examples are methyl cellulose, ethyl cellulose, hydroxypropyl cellulose, hydroxypropyl methylcellulose and sodium croscarmellose.

Preferred as release-modifying polymer are cellulose ether, preferably ethyl cellulose, preferably ethyl cellulose having an average molecular weight of 150,000 to 300,000 g/mol and/or an average degree of substitution, ranging from 1.8 to 3.0, preferably from 2.2 to 2.6. Alternatively preferred are cellulose ester, preferably cellulose acetate phthalate, carboxymethyl ethyl cellulose, hydroxypropyl methylcellulose phthalate and hydroxypropyl methylcellulose acetate succinate.

Polyvinyl polymers such as polyvinyl acetate or polyvinyl acetate copolymers, preferably polyvinyl acetate phthalate, and mixtures thereof can alternatively be used as release-modifying polymer.

More preferred are acrylic polymers or acrylic copolymers as release-modifying polymers, in particular acrylic polymers or acrylic copolymers.

Among acrylic polymers or acrylic copolymers, the release-modifying polymer is a polymer consisting of the structures according to the general formulae (1) and (2). 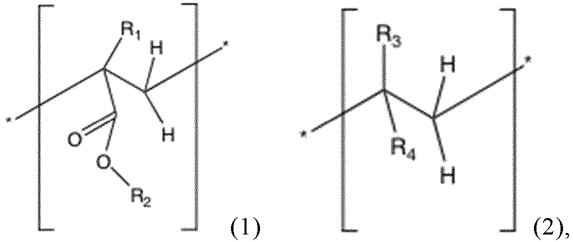 wherein in formulae (1) and (2)
R₁ is a hydrogen atom or a C₁-C₆ alkyl group, preferably a hydrogen atom or a methyl group or an ethyl group, particularly preferred a methyl group;
R₂ is a hydrogen atom or an alkyl group, preferably a hydrogen atom or a C₁-C₄ alkyl group, particularly preferred a methyl group, ethyl group or butyl group;
R₃ is a hydrogen atom or a C₁-C₄ alkyl group, preferably a hydrogen atom or a methyl group;
R₄ is a carboxylic acid or a derivative thereof, particularly preferred a group according to the formula -COOH or -COOR₅, wherein R₅ is an alkyl group or a substituted C₁-C₄ alkyl group, preferably a methyl, ethyl, propyl or butyl group, or -CH₂-CH₂-N(CH₃)₂ or -CH₂-CH₂-N(CH₃)₃⁺ halogen- (in particular Cl⁻) as substituted alkyl group.

The acrylic polymer according to formulae (1) and (2) is usually comprised of structures with a molar ratio of from 1 : 40 to 40 : 1. The preferred ratio of the structures of formula (1) to structures of formula (2) is from 2 : 1 to 1 : 1, particularly about 1:1. When R₄ is -COO-CH₂-CH₂-N(CH₃)₃⁺Cl⁻, the ratio of structures according to formula (1) to structures of formula (2) preferably is 20 : 1 to 40 : 1.

In case of an alternating copolymerization with a ratio of 1 : 1, this results in a preferred polymer according to formula (1+2)

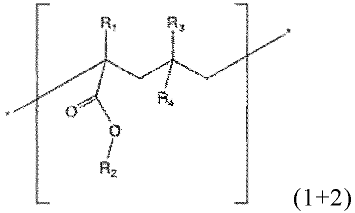

Polyacrylates according to formulae (1) and (2) as mentioned above are particularly preferred, wherein each R₁ is independently methyl or hydrogen, each R₂ is C₁-C₄ alkyl, preferably methyl or ethyl, R₃ is methyl or hydrogen, in particular methyl, and R₄ is -COO-CH₂-CH₂-N(CH₃)₃⁺Cl⁻. A particularly preferred ratio of the structures according to formula (1) to the structures according to formula (2) is about 40 : 1. A corresponding polymer preferably has a weight average molecular weight of from 50,000 to 250,000 g/mol, more preferred of from 120,000 to 180,000 g/mol. Such a copolymer is also referred to as Eudragit RS.

Polyacrylates according to formulae (1) and (2) as mentioned above are alternatively particularly preferred, wherein R₁, R₂ and R₃ are C₁-C₄ alkyl, particularly methyl, and R₄ is -COOH. A particularly preferred ratio of the structures according to formula (1) to the structures according to formula (2) is about 1 : 1. A corresponding polymer preferably has a weight average molecular weight of from 50,000 to 250,000 g/mol, more preferred of from 120,000 to 180,000 g/mol. Such a copolymer is also referred to as Eudragit L.

Especially preferred as release-modifying polymer is a mixture of the above-described copolymers Eudragit RS and Eudragit L. In a preferred embodiment the weight ratio (w/w) of Eudragit RS and Eudragit L is 7 : 1 to 1 : 7, preferably 6 : 1 to 1 : 4, more preferably 4 : 1 to 1 : 2, in particular 3 : 1 to 1 : 1, especially about 2:1.

It is preferred that statin and release-modifying polymer are present in particles (a) in a weight ratio (w/w) of 1 : 4 to 1 : 20, more preferably 1 : 5 to 1 : 18, even more preferably 1 : 6 to 1 : 15, in particular 1 : 8 to 1 : 12, especially about 1 : 10.

The particles (a) may comprise, apart from statin and release-modifying polymer, one or more pharmaceutically acceptable excipient(s). Pharmaceutical excipients such as solubilizers, glidants, disintegrants, surfactants and lubricants are known in the art. Suitable excipients are for example disclosed in "Lexikon der Hilfsstoffe für Pharmazie, Kosmetik und angrenzende Gebiete", published by H.P. Fielder, 4th Edition, and "Handbook of Pharmaceutical Excipients", 3rd Edition, published by A.H. Kibbe, American Pharmaceutical Association, Washington, USA, and Pharmaceutical Press, London. Preferably, particles (a) comprised by the present granulate do not comprise a further pharmaceutically acceptable excipient, i.e. it is preferred that the particles (a) comprised by the present granulate consist of statin and release-modifying polymer, preferably in the above-mentioned ratio.

Bovine serum albumin (BSA) can preferably be used as a solubilizer. Bovine serum albumin BSA is a protein which can be derived from cows. The full-length BSA precursor polypeptide has 607 amino acids, wherein an N-terminal 18-residue peptide is cut off from the precursor protein upon secretion such that the initial protein product contains 589 amino acid residues. An additional six amino acids are cleaved to yield the mature BSA protein that contains 583 amino acids.

The particles (a) may have a particle size from 10 µm to 1000 µm, preferably from 50 µm to 750 µm, in particular from 250 µm to 500 µm, determined by sieve analysis as described in the below experimental section.

The particles (a) may be prepared as known in the art. Suitable methods are for example co-precipitation, spray-drying, spray-congealing, hot-melt-extrusion and solvent evaporation also known as solvent cast. Further, the particles (a) may be obtained by 3D-printing methods.

Co-precipitation is a method in which the carrying down by a precipitate of substances normally soluble under the conditions employed can be achieved. The precipitate can subsequently be dried to obtain particles.

Spray drying is a method of forming particles from a liquid or slurry containing a solvent as well as statin and release-modifying polymer by rapidly drying with a hot gas. Examples of solvents are water, ethanol and mixtures thereof. Depending on the solvent and the sensitivity of the product, the hot gas can for example be air or nitrogen. When using spray-drying, fine particles are usually obtained.

Hot melt extrusion (HME) is a method in which a carrier material (in the present case release-modifying polymer) and preferably also a drug (in the present case: statin) are present and extruded in a molten state under specific conditions of pressure, velocity, and screw design of the extruder. Thereby the dispersion of the drug within the carrier is facilitated and, preferably, a non-crystalline form of the drug can be achieved. If necessary, the particle size of the extrudate can be reduced for example by cutting or milling.

Solvent evaporation or solvent cast is a method which is based on the evaporation of solvent(s) at a low temperature, often room temperature (which usually corresponds to 23°C). In the method a polymer is dissolved/dispersed in a first solvent and for example a drug is dissolved in a second solvent, wherein the first and the second solvent can be the same or different. Subsequently, the dissolution(s)/dispersion(s) are blended and afterwards the solvent or mixture of solvents is evaporated.

Spray congealing is a method to produce particles preferably without the use of organic or aqueous solvent. The molten slurry of agent and carrier or an emulsion of agent and carrier are sprayed via a heated nozzle in a cool airstream where the formation of the particles takes place.

The particles (a) containing statin and release-modifying polymer are preferably obtained by solvent evaporation. It is preferred that the particles (a) are obtained by the following steps:
(SE1) dissolving statin and optionally pharmaceutically acceptable excipient in a first solvent
(SE2) dissolving release-modifying polymer and optionally pharmaceutically acceptable excipient in a second solvent
(SE3) blending the solutions/dispersions from steps SE1 and SE2
(SE4) subjecting the blend from step SE3 to evaporation.

In step (SE1) statin, preferably statin in form of calcium salt, is dissolved, preferably completely dissolved, in a first solvent. Non-limiting examples of the first solvent are water and alcohols with 1 to 4 carbon atoms. More preferred are methanol and/or ethanol, in particular methanol. It is further preferred that step (SE1) is carried out under mechanical movement such as stirring. It is preferred that in step (SE1) no pharmaceutically acceptable excipient is added.

In step (SE2) release-modifying polymer is dissolved, preferably completely dissolved, in a second solvent. Non-limiting examples of solvent are C₁-C₆ alcohols. More preferred are methanol and/or ethanol. It is further preferred that step (SE2) is carried out under mechanical movement such as stirring. It is preferred that in step (SE2) no pharmaceutically acceptable excipient is added.

Steps (SE1) and (SE2) can be carried out in any order.

In step (SE3) the solutions/dispersions from steps SE1 and SE2 are blended. It is preferred that the solution from step (SE1) is added to the solution from step (SE2) under mechanical movement. The blending time is preferably from 1 to 30 minutes, more preferably from 2 to 25 minutes, even more preferably from 3 to 20 minutes, in particular about 10 minutes.

(SE4) subjecting the blend from step SE3 to evaporation. In preferred embodiment, the blend from step (SE3) is poured into a shallow vessel such as a silicone tray, a bowl, a cup or the like to enlarge the surface of the blend and thus to facilitate the evaporation of the solvent or mixture of solvents. After the evaporation, the resulting product can be cut or milled to obtain particles (a) containing statin and release-modifying polymer.

Steps (SE1), (SE2), (SE3) and (SE4) can independently be carried out at a temperature of from 10°C to 50°C, preferably from 15°C to 40°C, in particular from 20°C to 30°C, in particular at about 23°C (room temperature).

Apart from particles (a) containing statin and release-modifying polymer the granulate comprises binder (b).

A binder is generally a substance which is capable of increasing the strength of the resulting form, in the present case the granulate.

Suitable binders are for example polyvinylpyrrolidone, polyvinyl acetate, polyvinyl alcohol, methylcellulose, hydroxyethyl cellulose, hydroxypropyl cellulose, hydroxypropyl methylcellulose, carboxymethyl cellulose, cellulose glue sugars, pectin, dextran/dextrin, corn starch, gelatine, Gum Arabic, alginate and its salts, tragacanth or mixtures thereof. The binder (b) is preferably selected from gelatine, cellulose glue, Gum Arabic, methylcellulose, carboxymethyl cellulose, hydroxyethyl cellulose, dextrin, alginate and its salts, pectin, tragacanth, or mixtures thereof, more preferably from gelatine, methylcellulose, hydroxyethyl cellulose, dextrin, alginate and its salts, pectin, tragacanth, or mixtures thereof. It is particularly preferred that the binder (b) is alginate and/or its salts, especially a salt of alginate, more especially alginate in form of its sodium salt.

Particles (a) containing statin and release-modifying polymer and binder (b) are preferably present in a weight ratio (w/w) of 3 : 1 to 1 : 3, more preferably 2 : 1 to 1 : 2, even more preferably 1.5 : 1 to 1 : 1.5, in particular about 1 : 1.

The average particle size of the granulate is preferably between 0.1 to 1.5 mm, more preferably between 0.4 to 1.3 mm, even more preferably between 0.6 and 1.2 mm, in particular about 1 mm as determined by sieve analysis as described in the below experimental section.

A method for preparing the granulate can preferably comprise the steps of
(i) preparing particles (a) containing statin and release-modifying polymer,
(ii) granulating the particles of step (i) with binder (b) to obtain the granulate.

As far as step (i) is concerned, the particles (a) containing statin and release-modifying polymer can be prepared as described above. It is preferred that the particles are prepared by steps (SE1) to (SE4) as described above.

In step (ii) the particles of step (i) are granulated with binder (b) to obtain the present granulate.

The granulate can preferably be obtained either by a wet or a dry granulation process as known in the art. "Granulating" is generally understood to mean the formation of relatively coarse granulate as a solid by assembling and/or aggregating finer powder particles (agglomerate formation, or build-up granulation). If necessary, the size of granulate can be reduced to a predetermined size, for example by milling.

The granulate can preferably be obtained either by a wet or a dry granulation process, preferably the granulate according to the invention is obtained by a wet granulation process.

Apart from the component(s) to be granulated, a granulation liquid is used in a wet granulation. Non-limiting examples of suitable granulation liquids are water, methanol, ethanol and mixtures thereof. In a preferred embodiment an aqueous granulation liquid is used. Generally, "aqueous" means that the granulation liquid contains substantial amounts of water. Preferably, the granulation liquid is water, more preferably from 80 to 100 wt.-% water, especially 90 to 100 wt.-% water, based on the total weight of the granulation liquid. The solvent of the granulation liquid preferably consists essentially of water, in particular consists of water.

The granulate can preferably be obtained by a fluid bed granulation as known in the art.

Alternatively, the granulate can preferably be obtained by wet granulation comprising the steps of
(WE1) homogenizing particles (a) containing statin and release-modifying polymer and binder (b),
(WE2) addition of the granulation liquid, and again homogenizing the resulting mixture,
(WE3) comminuting the mixture of step (WE2) to obtain a granulate
(WE4) drying the granulate obtained from step (WE3).

As far as particles (a) containing statin and release-modifying polymer and binder (b) are concerned, the same applies as described above.

Homogenizing in step (WE1) can be done by techniques known in the art such as milling or subjecting the particles (a) containing statin and release-modifying polymer and binder (b) to a mixing process. Particles (a) containing statin and release-modifying polymer and binder (b) are preferably homogenized in a mortar with a pestle.

In step (WE2), granulation liquid is added to the homogenized product of step (WE1) and the resulting mixture is homogenized again. Non-limiting examples of the granulation liquid are water and alcohols with 1 to 4 carbon atoms, in particular water. It is preferred that homogenizing the resulting mixture is also carried out in a mortar with a pestle.

In step (WE3), the mixture of step (WE2) is comminuted to obtain a granulate. Comminuting can be carried out for example by milling, cutting or extruding. In a preferred embodiment the mixture of step (WE2) is extruded through a sieve, i.e. the mixture of step (WE2) is pressed through a sieve preferably with a mesh size such that the resulting granulate has an average particle size as described above.

In step (WE4), the granulate obtained from step (WE3) is dried. Drying is known in the art. Drying can for example be carried out at about 25°C without reduced pressure. Drying is preferably carried out until weight constancy of the product to be dried is achieved. In a preferred embodiment, drying can be carried out for 5 minutes to 48 hours, preferably for 3 hours to 36 hours, more preferably for 6 to 30 hours, in particular for about 24 hours. In a preferred embodiment, the granulate obtained from step (WE3) is dried for about 24 hours at 25°C without reduced pressure.

The first statin-containing composition provides modified release of statin.

Within the present application, generally the term "modified release" is used as defined by the USP. Preferably, modified release forms are those whose drug release characteristics accomplish therapeutic or convenience objectives not offered by immediate release forms. Generally, immediate release (IR) forms release at least 70 % of the drug within 1 hour or less. The term "modified release" can comprise delayed release, prolonged release, sustained release, controlled release and/or extended release.

Delayed release usually indicates that statin is not being released immediately after administration but at a later time, preferably less than 10% are released within two hours after administration.

Prolonged release usually indicates that statin is provided for absorption over a longer period of time than IR forms, preferably for about 2 to 24 hours, in particular for 3 to 12 hours.

Sustained release usually indicates an initial release of drug statin, sufficient to provide a therapeutic dose soon after administration, preferably within two hours after administration, and then a gradual release over an extended period of time, preferably about 3 to 18 hours, in particular 4 to 8 hours.

Controlled release dosage forms usually release the statin at a constant rate and provide tissue concentrations that remain essentially invariant with time.

Extended release usually indicates a slow drug statin release, so that tissue concentrations are maintained at a therapeutic level for a time period of between 6 and 72 hours, preferably between 8 and 72 hours.

In a preferred embodiment according to the invention, the modified release of statin provided by the first composition is an extended release.

In a preferred embodiment according to the present invention, the release of statin provided by the first composition is
- 0 to 20 %, preferably 1% to 15%, more preferably 3% to 10% after 1 hour,
- 20% to 70%, preferably 30% to 65%, more preferably 40 to 60% after 8 hours,
- 40% to 90%, preferably 55% to 85%, more preferably 60% to 80% after 24 hours,
- 60% to 98%, preferably 70% to 96%, more preferably 80% to 95% after 48 hours, and/or
- 75% to 100%, preferably 80% to 100%, more preferably 95% to 100% after 72 hours,
as measured in a Franz cell as described in the below experimental section.

In a preferred embodiment according to the invention, a second statin-containing composition is applied directly into the wound, wherein said second composition provides immediate release of statin.

It is preferred that the second composition comprises, apart from stain, a filler.

The solid composition contained in the second dosage form further comprises a filler.

A filler generally means substances which serve to form a composition or a dosage form in the case of small amounts of pharmaceutically active agent (e.g. less than 30% by weight). This means that fillers "dilute" the pharmaceutically active agent(s) in order to produce a mixture which can be adequately handled. The normal purpose of fillers therefore is to obtain a suitable composition or dosage form size.

Examples of fillers are lactose, lactose derivatives, starch, starch derivatives, treated starch, chitin, cellulose and derivatives thereof, calcium phosphate, calcium hydrogen phosphate, sucrose, calcium carbonate, magnesium carbonate, magnesium oxide, maltodextrin, calcium sulphate, dextran, dextrin and/or dextrose, hydrogenated vegetable oil and mixtures thereof. Preferred are lactose, lactose derivatives, starch, starch derivatives, microcrystalline cellulose, cellulose and derivatives thereof, and mixtures thereof. More preferred is lactose, microcrystalline cellulose, cellulose and derivatives thereof, and mixtures thereof, in particular lactose, microcrystalline cellulose, cellulose derivatives and mixtures thereof, especially microcrystalline cellulose, lactose and mixtures thereof, in particular lactose.

The statin contained in the second composition can be present in a crystalline or a non-crystalline form. The statin contained in the second composition is preferably present in crystalline form. Such a composition may be obtained by blending statin and filler in a mixing device for example under mechanical stirring.

In a preferred embodiment, the statin contained in the first composition is present in non-crystalline form and the statin contained in the second is present in crystalline form.

In an alternative preferred embodiment, the statin contained in the second composition is present in a non-crystalline form. Such a solid composition may be obtained by spray-drying statin and filler.

Preferably, statin and filler contained in the second composition are present in a weight ratio (w/w) of between 1 : 3 and 1 : 20, preferably between 1 : 5 and 1:15, more preferably between 1 : 7 to 1 : 12, in particular about 1 : 9.

Alternatively preferred, the content of statin is 5 to 30% by weight, preferably 6.5 to 20% by weight, more preferably 8.5 to 15% by weight, in particular 10% by weight based on the weight of the sum of statin and filler contained in the second composition.

The weight ratio (w/w) of the first composition to the second composition is preferably from 1 : 1 to 30 : 1, preferably from 2 : 1 to 20 : 1, more preferably from 3 : 1 to 15 : 1.

Alternatively preferred, the weight ratio (w/w) of statin contained in the first composition to statin contained in the second composition is preferably from 10:10 to 95 : 5, preferably 20 : 10 to 90 : 10 , more preferably 70 : 30 to 85 : 15.

In a preferred embodiment according to the present invention, the release of statin provided by the combined first and second compositions is preferably a bi or more phasic release, in particular a biphasic release.

A bi or more phasic release can be regarded as a release wherein one part of the release per time is higher that the release per time of at least one other part. The release per time within the first 24 hours is preferably higher than the release per time from the 24^{th} hour to the 72^{th} hour. The release of statin provided by the combined first and second compositions is preferably 60 to 95% after 24 hours and 95 to 100%, preferably 100% after 72 hours. In other words, the release after 24 hours is 60 to 95%, while the release between the 24^{th} hour to the 72^{th} hour is 5 to 40% to sum up to preferably 100%.

In an alternative preferred embodiment according to the present invention, the release of statin provided by the combined first and second compositions is
- 15 to 50%, preferably 17% to 35%, more preferably 20% to 40% after 1 hour,
- 30% to 75%, preferably 45% to 72%, more preferably 60 to 70% after 8 hours,
- 60% to 95%, preferably 70% to 95%, more preferably 80% to 95% after 24 hours,
- 70% to 100%, preferably 80 to 99%, more preferably 90% to 99% after 48 hours, and/or
- 80% to 100%, preferably 90 to 100%, more preferably 95 to 100% after 72 hours,
as measured in a Franz cell as described in the below experimental section.

Statins are known in the art. The term "statin" as used in the present application can refer to free statin as well as to its pharmaceutically acceptable salts, hydrates, solvates, polymorphs and mixtures thereof.

Non-limiting examples for statin are atorvastatin, fluvastatin, lovastatin, mevastatin, pitavastatin, pravastatin, rosuvastatin, simvastatin or mixtures thereof. Preferred is atorvastatin, preferably in form of a salt, more preferably atorvastatin in form of its calcium salt, in short atorvastatin calcium.

Applying the first composition and optionally the second composition directly into the wound can be carried out as known in the art. Preferably, it is carried out by sprinkling the first composition or the first and second compositions into the wound.

In a preferred embodiment according to the present invention, the amount of statin applied into the wound is 0.01 to 10 mg/cm², preferably 0.02 to 7.5 mg/cm², more preferably 0.03 to 5.0 mg/cm², in particular about 2.5 mg/cm², especially about 0.1 g/cm² of wound tissue.

In a preferred embodiment according to the invention, the amount of statin applied into the wound leads to a statin concentration in the wound tissue which is as least as high as the statin concentration in the wound tissue after oral administration of the recommended statin dosage for treating dyslipidemias. It is preferred that the statin concentration in the wound tissue resulting from the administration of the first composition or the first and second compositions is twice to 10 times, preferably about three times, higher than the statin concentration in the wound tissue after oral administration of the recommended statin dosage for treating dyslipidemias.

In a preferred embodiment according to the invention, the first statin-containing composition or the first and second statin-containing compositions can be administered from four times a day to every three days. It is preferred that the administration is every two days. It is more preferred that the administration is every three days. More preferably, the administration is two or three times per week.

Further, the invention relates to a method for treating a wound with impaired wound healing comprising applying statin directly into the wound, wherein statin contained in a first solid composition and wherein said first composition provides modified release of statin. With regard to the method for treating a wound with impaired wound healing, the same as for the statin for use in the treatment of a wound with impaired healing applies. Optionally, a wound dressing can be further applied over the wound. Wound dressings to be applied over the wound are known in the art. It is preferred that the wound dressing is sterile. Further applying a wound dressing, preferably a sterile wound dressing, bears the advantage that the wound stain as described above is applied can be prevented from being contaminated by any pollutants and germs present in the environment.

### Experimental section

### 1. Analytical part:

### 1.1 Determination of release

Dissolution testing represents crucial evaluation in dosage form development. It observes drug release over a defined time interval in a defined dissolution medium. The topical administration and wound treatment represent compartments with limited fluid volume. Therefore, to simulate the desired administration, a non-pharmacopeial Franz cell diffusion system with 20 ml of medium was employed. The cell was used as a vessel, no membrane or donor chamber was employed. A cap was placed at the top of the acceptor chamber to minimize water evaporation.

Specifications of Franz cells setup were as follows:
- Franz cells with diffusion area of 4.91 cm² (PermeGear, Inc., USA)
- Heating system Julabo F30-C (JULABO GmbH, Germany)

To simulate natural tissue pH environment, a phosphate buffer solution of pH 7.4 was used. The buffer was prepared by dissolving 0.6 g potassium dihydrogen phosphate, 6.4 g disodium hydrogen phosphate dodecahydrate and 5.9 g sodium chloride in 900 ml water under stirring, and then diluted with water up to 1000 ml.

Sample preparation: Each cell was filled with 20 ml of the pH 7.4 phosphate buffer solution which was tempered to 37°C by the heating system. No stirring was performed to correspond with minimal instantaneous fluid movement in wounds. An accurately weighed portion of sample (equivalent to 1 mg atorvastatin calcium) was weighed out and transferred onto the medium surface in the cell with no further manipulation. At specific sampling times (30 minutes, 1, 2, 3, 5, 8, 24, 48, and 72 hours) 1 ml of the medium was taken from the area under the sample using 1 ml syringe with lumbar needle, and 1 ml of fresh PBS was added through the port to the cell. The taken samples were filtered (pore size 0.45 µm) into vials and stored in a refrigerator before HPLC analysis.

HPLC analysis:
- Agilent 1260 (Agilent Technologies, Santa Clara, CA, US)
- Column: 250 × 4.6 µm BDS Hypersil C18
- Mobile phase: Acetonitrile:phosphoric acid (0.02M) 60:40
- Injection volume: 5 µL
- Flow rate: 1 ml/min
- Detector wavelength: 248 nm

Calibration solution: An accurately weighed portion of atorvastatin calcium (10 mg) was weighted out and transferred to a volumetric flask of 100 ml (concentration of 0.1 mg/ml) and dissolved in phosphate buffer solution (pH 7.4). This stock solution was subjected to an ultrasound treatment for at least 30 minutes and left standing for 12-24 hours. Several calibration solutions for calibration curve construction were prepared (different concentrations - 0.08 mg/ml, 0.06 mg/ml, 0.04 mg/ml, 0.02 mg/ml) from the stock solution. 1 ml of each calibration solution was filtered into vial prior to HPLC measurement.

The same HPLC method was used to measure the collected samples. The results were recalculated to obtain a cumulative drug release graph.

### 1.2 Determination of average particle size by sieve analysis

Sieve analysis is traditionally utilized to determine particle size distribution or separation of different size fractions. A sieve stack usually consists of seven sieves stacked on top of each other with a decreasing mesh size. Suitable test sieves conform to the most current edition of International Organization for Standardization Specification ISO 3310-1: Test Sieves - Technical Requirements and Testing. A nest of sieves having a √2 progression of the area of the sieve openings is recommended. The sample is placed on the top sieve. Sieving mediated by vibrations continues until the mass of the sample on each sieve no longer changes (= constant mass). Each sieve is weighted, and the volume of each fraction is calculated as a percentage by weight, resulting in a mass-based distribution.

In this experiment, the sieve analysis was used to separate different size fractions of the crushed polyacrylate films. Sieves with mesh sizes (apertures) of 500 and 250 µm were stocked on the top of a collecting vessel and placed on a vibrating desk of a Retsch AS 200 basic sieve analysis device. The powder was placed onto the largest sieve, the sieve set was covered with a lid and secured with clamps to prevent the sieve column from shifting. The sieving process was carried out for 10 minutes at an amplitude of 60. The particles were separated into three size fractions: < 250 µm, 250-500 µm and > 500 µm. The fraction of 250-500 µm was used for further granulate preparation.

### 2. Examples

### 2.1 Preparation of particles containing statin and release-modifying polymer

Dispersions of Eudragit were obtained by weighing 20 g of Eudragit polymer (Eudragit^{®} RS and Eudragit^{®} L in different ratios) in a beaker and adding 80 g of ethanol and subsequent homogenizing via a stirring rod. 2.0 g of atorvastatin calcium were dissolved in 250 ml methanol using a magnetic stirrer. The resulting atorvastatin-containing solution was mixed with 90 g of the Eudragit-containing dispersion under stirring and the resulting dispersion was cast on a silicone baking mat and left to dry at ambient laboratory conditions to obtain an atorvastatin/Eudragit-containing film. The film was manually crushed in a mortar with a pestle to obtain particles containing 10% atorvastatin and 90% Eudragit. The particles can be separated by sieving with one or more sieves with the corresponding mesh sizes into fractions of different sizes such as 125 to 250 µm or 250 to 500 µm.

### 2.2 Preparation of the first composition containing statin, release-modifying polymer and binder

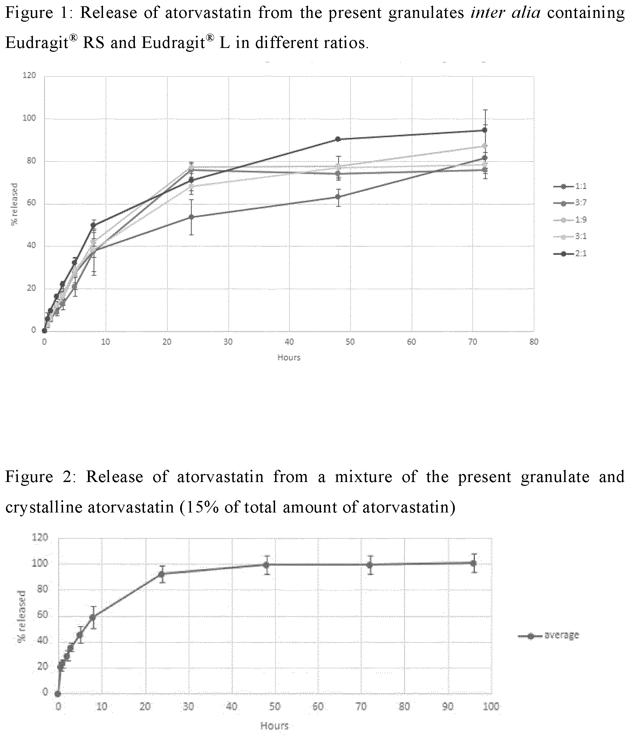

Particles obtained by Example 2.1 and alginate sodium in a weight ratio (w/w) of 1 : 1 were weighed in a mortar and homogenized with a pestle. The mixture was then sprinkled with water (mixture to water in a ratio of 2 : 1) and again thoroughly homogenized. Subsequently, the resulting sticky mixture was pressed (extruded) through a sieve with a pore size of 1 mm to obtain the granulate. The granulate was further allowed to dry for 24 hours at ambient conditions. The release profiles of atorvastatin from the granulates comprising particles having different weight ratios of Eudragit^{®} RS and Eudragit^{®} L and alginate sodium are shown in Figure 1.

### 2.2.1 Granulate plus crystalline atorvastatin

**2.2.1. A** To 17 mg of the first composition according to Example 2.2 having a ratio of Eudragit^{®} RS and Eudragit^{®} L of 2 : 1 are added 0.15 mg of crystalline atorvastatin and the mixture was homogenized. The corresponding release profile is shown in Figure 2.

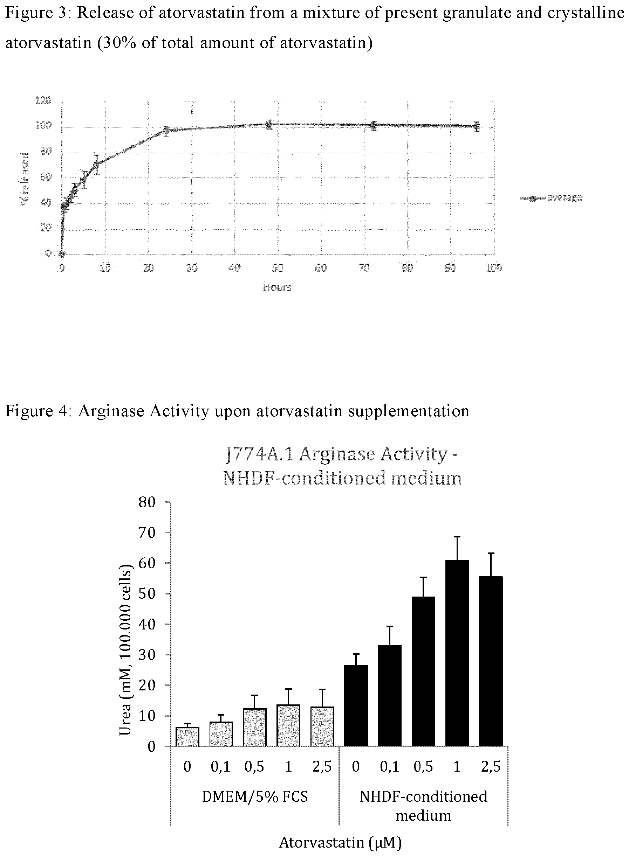

**2.2.1.B** To 14 mg of the first composition according to Example 2.2 having a ratio of Eudragit^{®} RS and Eudragit^{®} L of 2 : 1 are added 0.30 mg of crystalline atorvastatin and the mixture was homogenized. The corresponding release profile is shown in Figure 3.

### 2.3 Preparation of the second composition comprising statin and filler

**2.3.1** 4.05 g of lactose and 0.3 g of atorvastatin are thoroughly blended for 30 min in a conventional mixing device.

**2.3.2** 2.7 g of hydroxypropyl methylcellulose with a median particle size of 100 µm and a molecular weight of less than 100 kDa (Hypromellose K100 Premium LVCR) were suspended/dissolved in 87.3 g of water. Subsequently 0.3 g of atorvastatin were added, and the resulting suspension was spray-dried under the following conditions:

| | |
|---|---|
| Inlet temperature: | 160°C |
| Out let temperature: | 80°C |
| Atomization pressure: | 2 bars |
| Air speed: | 3.5 m/s |
| Pump speed: | 1665 ml/h |
| Nozzle size | 2 mm |

### 3. Expression of Arginase Activity

Macrophages are key regulators in tissue repair. They fight invading microorganisms (together with neutrophil granulocytes, PMN), degrade non-viable tissue and orchestrate the wound healing response. In the extreme, macrophages are defined as pro-inflammatory (M1 phenotype, e.g. when fighting bacteria or when stimulated with bacterial products/break-down products such as lipopolysaccharides for gram-negative bacteria). In the other extreme, and mostly in murine animal models, the M2 phenotype was described as driving the resolution of inflammation and stimulating tissue repair (angiogenesis, granulation tissue formation) (Ferrante CJ, Leibovich SJ. (2012). Regulation of macrophage polarization and wound healing. Adv Wound Care 1: 10-16). These phenotypic descriptions are over-simplistic in humans and human disease but have greatly helped to understand the underlying processes in tissue repair.

For wound healing eventually, the "M2" macrophage phenotype is desired for the resolution of tissue-damaging inflammation, the induction of angiogenesis, and the activation of mesenchymal cells to produce extracellular matrix. Again, in humans, but in many experimental animal models too, the exact definition of macrophage phenotypes is overlapping (Xue J, Schmidt SV, Sander J, Draffehn A, Krebs W, Quester I, De Nardo D, Gohel TD, Emde M, Schmidleithner L, Ganesan H, Nino-Castro A, Mallmann MR, Labzin L, Theis H, Kraut M, Beyer M, Latz E, Freeman TC, Ulas T, Schultze JL. (2014) Transcriptome-based network analysis reveals a spectrum model of human macrophage activation. Immunity 40:274-88). Still, conceptually the phenotype of M2 macrophages in rodents is considered to reflect the underlying mechanisms in other species. Arginase expression is a key feature of alternatively activated macrophages (M2 phenotype), and has been studied in wound healing (Campbell L, Saville CR, Murray PJ, Cruickshank SM, Hardman MJ. (2013)). Local arginase 1 activity is required for cutaneous wound healing. J Invest Dermatol 133, 2461-2470). After 5 days arginase-1 expression reached its maximum in these experiments.

In a murine macrophage cell line, J774A. 1 cells (Ralph P, Moore MA, Nilsson K. (1976) Lysozyme synthesis by established human and murine histiocytic lymphoma cell lines J Exp Med 143:1528-33), culture in normal human fibroblast-conditioned medium (DMEM/5% FCS) resulted in the expression of arginase enzyme activity. This expression was further enhanced by the presence of atorvastatin reaching a maximum at 2.5 µM (cf. right side of Figure 4 with dark black bars).

In controls, cultured in DMEM/5% FCS (without fibroblast pre-conditioning) the baseline induction of arginase enzyme activity was missing and the superimposed effects of atorvastatin on arginase enzyme activity were virtually absent.

Systemic concentrations of 2.5 µM atorvastatin are difficult if not impossible to achieve in routine clinical practice by systemic application of atorvastatin (per os, i.v.). Topical application of atorvastatin can achieve high doses which are diluted in the organism, which is confirmed by the FDA Report (Atorvastatin New Drug Application 1996). In wounds, local treatment with wound dressings usually relies on 2-day or 3-day dressing change intervals (if not longer) which make frequent atorvastatin applications (e.g. daily) impracticable or impossible.

### 3.1 Methods

J774A.1 cells (DSMZ, ACC-170, https://celldive.dsmz.de/celllines/J-774A.1) were cultured to 80% confluence. Medium changes were done daily at high cell densities.

50.000 cells/96-well were seeded in 96-well plates. After attachment medium was changed the next day and replaced by DMEM/5% FCS (controls) or by normal human fibroblast-conditioned DMEM/5% FCS (conditioned for 3 days) diluted 1:1 with fresh DMEM/5% FCS. Medium changes were daily and non-adherent cells were removed at the medium changes. Atorvastatin was supplemented in both conditions from a 10 mM stock in methanol and subsequent dilution steps in DMEM/5% FCS to give the advertised concentrations in Figure 4.

Cells were cultured for 4 days with or without atorvastatin influence. Cell harvesting and arginase activity determinations were done essentially according to Corraliza et al. (Corraliza IM, Campo ML, Soler G, Modolell M. (1994). Determination of arginase activity in macrophages: a micromethod. J Immunol Meth 174:231-235).

Cells were lysed in 50 µl in lysis buffer (0.1% Triton X-100, Tris/HCl, pH 7.5) and left at room temperature for 10 min. 50 µl 10 mM MnCl₂ were added, and the plate was incubated at 55°C for 10 min. to activate the enzyme. In these experiments 12 µl of the lysate were diluted with 13 µl dilution buffer (0.05% Triton X-100, Tris/HCl, pH 7.5, 5 mM MnCl₂) and 25 µl 0.5 M arginine, pH 9.7 were added. The mix was incubated at 37°C for 60 min. and the reaction was stopped by the addition of an acid mixture (1 vol. H₂SO₄, 3 vol. H₃PO₄, 7 vol. H₂O). 25 µl 9% α-isonitrosopropiophenone (dissolved in 100% ethanol) were added and the reaction mix was incubated at 95°C for 45 min. The mix was left to cool for 10 min. to room temperature. 100 µl were transferred to a 96-wells and quantified by measuring the absorption at 540 nm in a Tecan infinite 200Pro (Firmware: V_3.22_12/10_Infinite (Dec 14 2010/13.07.14)) ELISA plate reader.

Fig. 4 illustrates the effect of different atorvastatin concentrations on arginase enzyme activity in the J774A.1 macrophage cell line as a proxy of the M2 macrophage phenotype. After 4 days, J774A.1 cells did not express noticeable arginase enzyme activity when cultured in plain DMEM/5% FCS and in the absence of atorvastatin (grey bars). At 1.0 and 2.5 µM arginase activity was slightly induced (grey bars).

When J774A.1 cells were grown in fibroblast conditioned medium (DMEM/5% FCS, without atorvastatin) there was a massive increase in arginase enzyme activity. This increase was further stimulated with higher doses of atorvastatin, 0.5 µM, 1,0 µM and 2.5 µM.

These results suggest that high doses of atorvastatin strongly increase arginase enzyme activity - a marker of M2 macrophage polarization - once the cells receive the correct set of stimuli (fibroblast-conditioned DMEM/5% FCS vs. DMEM/5 %FCS). And these results further suggest that the concentrations required are higher than doses typically achieved with systemic treatment dosing schedules.

## Claims

1. Statin for use in the treatment of a wound with impaired healing, wherein a first solid statin-containing composition is applied directly into the wound, and wherein said first composition provides modified release of statin.

2. Statin for use according to claim 1, wherein the treatment comprises the activation of macrophages in the wound and/or angiogenesis and/or epithelialization.

3. Statin for use according to claim 1 or 2, wherein the first statin-containing composition is in granular form.

4. Statin for use according to any one of claims 1 to 3 wherein the modified release is an extended release of statin.

5. Statin for use according to any one of claims 1 - 4, wherein the release of statin is 0% - 20% after 1 hour, 20% - 70% after 8 hours, 40% - 90% after 24 hours, 60% - 98% after 48 hours and/or 75% - 100% after 72 hours, as measured in a Franz cell.

6. Statin for use according to any one of claims 1 to 5, wherein a second solid statin-containing composition is applied directly into the wound, wherein said second composition provides immediate release of statin.

7. Statin for use according to claim 6, wherein the release of statin provided by the combined first and second compositions is a biphasic release.

8. Statin for use according to claim 6 or 7, wherein the release of statin provided by the first and the second solid compositions is 15% - 50% after 1 hour, 30% - 75% after 8 hours, 60% - 95% after 24 hours, 70% - 100% after 48 hours and/or 80% - 100% after 72 hours, as measured in a Franz cell.

9. Statin for use according to any one of claims 1 to 8, wherein the statin is atorvastatin.

10. Statin for use according to any one of claims 1 to 9, wherein the total amount of statins administered into the wound is 0.01 - 10 mg per cm² of wound tissue.

11. Statin for use according to any one of claims 1 to 10, wherein the amount of statin applied into the wound leads to a statin concentration in the wound tissue which is as least as high as the statin concentration in the wound tissue after oral administration of the recommended statin dosage for treating dyslipidemias.

12. Statin for use according to claim 11, wherein the amount of statin applied into the wound leads to a statin concentration in the wound tissue which is twice to 10 times as least as high as the statin concentration in the wound tissue after oral administration of the recommended statin dosage for treating dyslipidemias.

13. Statin for use according to any one of claims 1 to 12, wherein the first statin-containing composition or the first and second statin-containing compositions can be administered from four times a day to every three days.

14. Statin for use according to claim 13, wherein the administration is every two days or every three days.

15. Method for treating a wound with impaired healing comprising applying statin directly into the wound, wherein the statin is contained in a first solid composition and wherein said first composition provides modified release of statin.
